# EUROPEAN PATENT APPLICATION

(11) **EP 1 911 741 A1**
(43) Date of publication of application: **16.04.2008**
(21) Application number: 05781841.1
(22) Date of filing: 29.08.2005
(51) Int. Cl.: C07C 327/24, C07C 327/02, C07J 31/00

(54) **METHOD FOR THE PREPARATION OF FLUTICASONE PROPIONATE**

(30) Priority: 26.07.2005 CN 200510028147
(71) Applicant: Shanghai Aurisco International Trading Co., Ltd., Jing'an District Shanghai 200-042 (CN)
(72) Inventor: CHU, Dingjun, Zhejiang 317200 (CN); ZHANG, Defa, Zhejiang 317200 (CN)
(74) Representative: Korga, Leokadia
(86) International application number: PCT/CN2005/001339
(87) International publication number: WO 2007/012228

(57) **Abstract**

Taught is a method for preparing S-fluoromethyl-6α,9α- difluroro-11β-hydroxy-16α-methyl-17α- propionyloxy-3-oxoandrosta-1,4-diene-17β-carbothioate (fluticasone propionate). The present method is simple, convenient, and mild, yields highly pure product, and is suitable for use commercially on a large scale.

## Description

### FIELD OF THE INVENTION

The invention relates to the field of pharmaceuticals, and specifically to a method for preparation of fluticasone propionate.

### BACKGROUND OF THE INVENTION

A process for preparing fluticasone propionate, the compound of formula **[1]**, has heretofor been described in U.S. Patent 4,335,121.

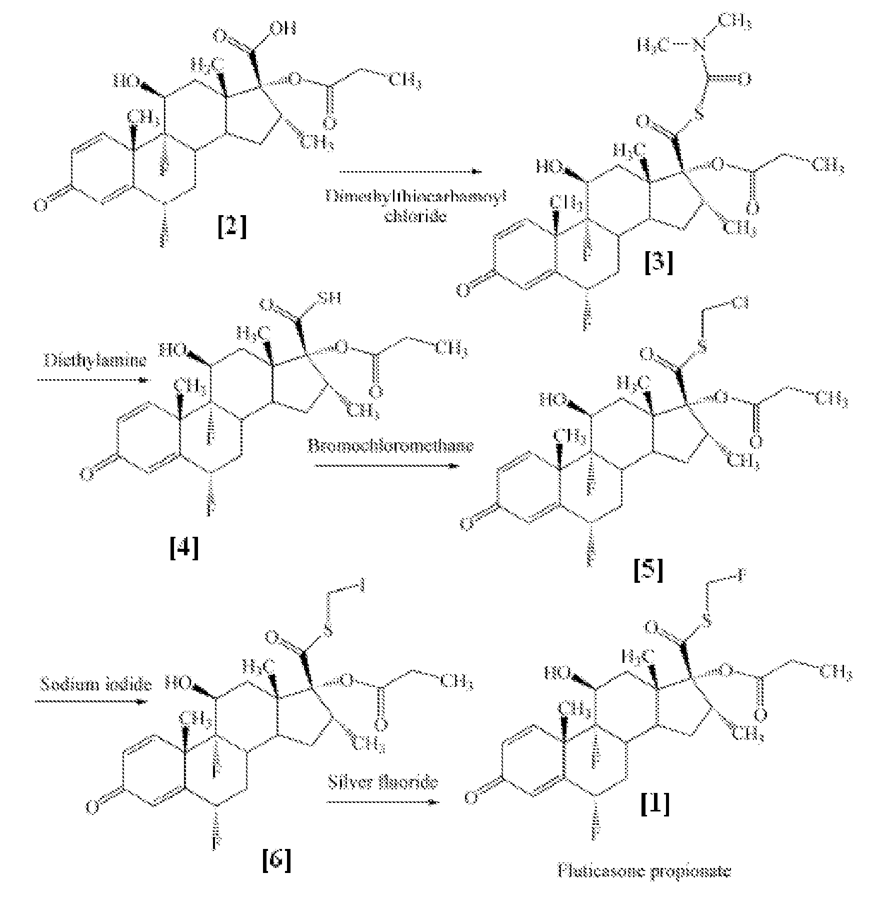

The process starts with the reaction of compound **[2]** with N,N-dimethyl thiocarbamoyl chloride to obtain compound **[3]**; refluxing it in the presence of diethylamine to obtain compound **[4]**, which is then reacted with bromochloromethane, followed by displacement with sodium iodide and silver fluoride to obtain compound **[1]**, fluticasone propionate. This process is complicated and the synthetic route is long. The yield is accordingly low, while the cost is high and uses an expensive reagent, silver fluoride. Therefore, this process isn't useful commercially.

Similarly, in the Intl. Pat. Publ. No. WO 01/62722 (which corresponds to U.S. Pat. Appl. Publ. No. 2002/0133032), compound **[2]** is reacted with dimethylthiocarbamic chloride in butanone in the presence of catalysts, sodium iodide and triethylamine to yield compound **[3]**, which is then reacted with sodium hydrosulphide to yield compund **[4]**, which is finally reacted with fluorochloromethane to yield compound **[1]**. Since excess sodium hydrosulphide is turned into hydrogen sulfide and a large amount of fluorochloromethane is employed, the process is environmentally unfriendly. Meanwhile, impurities resulting from using sodium hydrosulphide to yield sulfoacid **[4]** are hard to remove from the reaction mixture, which makes it difficult to improve the quality of the final product. The cost is also high.

### BRIEF SUMMARY OF THE INVENTION

This invention relates to a simple, efficient and economical method to produce fluticasone propionate on a commercial scale.

This invention provides a method for preparing S-fluoromethyl-6α,9α- difluroro-11β-hydroxy-16α-methyl-17α-propionyloxy-3-oxoandrosta-1,4-diene-17β-carbothioate (fluticasone propionate, compound **[1]**).

The present invention includes the following steps:

(1) compound **[2]**, 6α,9α-difluroro-11β-hydroxy-16α-methyl-17α-propionyloxy-3- oxoandrosta-1,4-diene-17β-carboxylic acid, is reacted with N,N-dimethyl thiocarbamoyl chloride in the presence of catalyst, 4-dimethylaminopyridine, in a ketonic or ethereal solvent, such as butanone or tetrahydrofuran, at between 0 and 40°C to yield compound **[3]**;

(2) compound **[3]**, 17β-((N,N-dimethyl-carbamyl)-thio) formoxyl-6α,9α-difluroro-11β-hydroxyl-16α-methyl-17α-propionyloxy-3-oxoandrosta-1,4-diene is decarboxamidated in a reaction system comprising an alcohol and a phosphate under alkaline reaction conditions to yield an alkali salt of compound [**4]**, i.e., compound **[4a]**; the alkali salt is neutralized with acid to obtain compound **[4]**;

(3) fluorobromomethane is first reacted to form a complex with an organic base in a solvent to activate the fluoromethyl group and then reacted with 6α,9α-difluroro-11β-hydroxyl-16α-methyl-17α-propionyloxy-3-oxoandrosta-1,4-diene-17β-thiocarboxylic acid (compound **[4]**) to yield compound **[1]** in a fluoromethylation reaction.

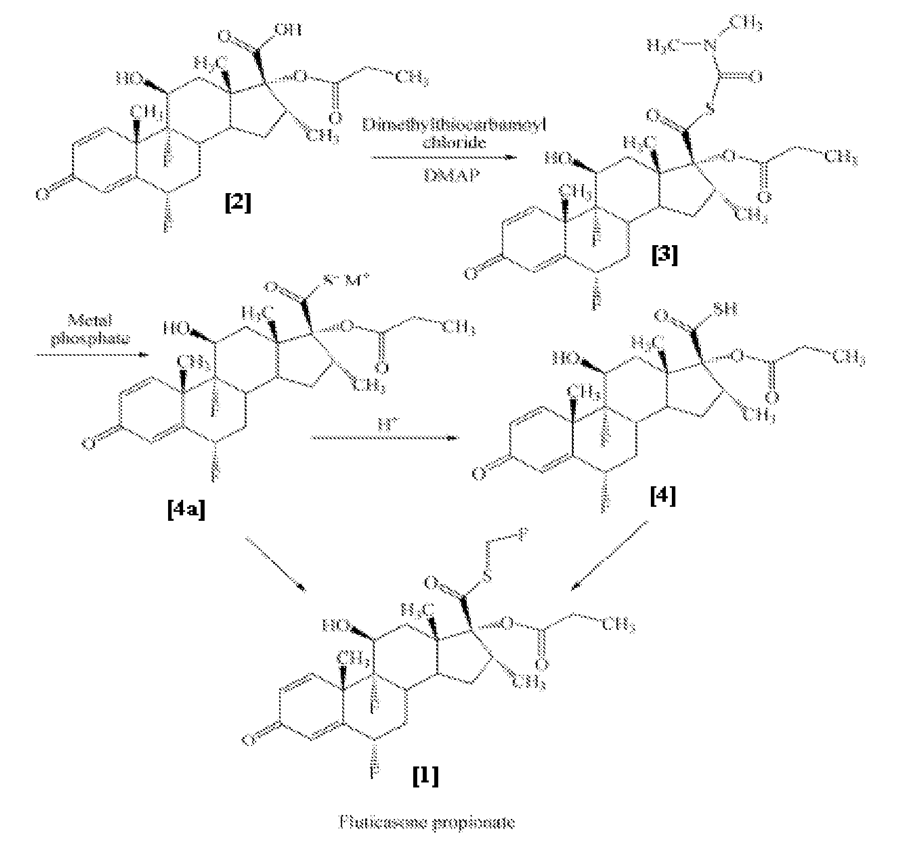

The present invention has the following characteristics:

(a): The catalyst, sodium iodide or other iodides is not used for the preparation of compound **[3]** from compound **[2]**. The acid absorbent, 4-dimethylaminopyridine, is used instead, and compound **[2]**, 6α, 9α-difluroro-11β-hydroxy-16α-methyl-17α-propionyloxy-3-oxoandrosta-1,4-diene-17β-carboxylic acid, is reacted with dimethyl thiocarbamoyl chloride for 2-3 hours at between 0 and 40°C, and preferably at between 25 and 35 °C, in a ketonic or ethereal solvent, such as butanone or tetrahydrofuran. Dimethyl acetamide and water are added, and then the temperature is cooled to between 0 and 5 °C to obtain compound **[3]** in a good quality.

(b): In the preparation of compound **[4]** from compound **[3]**, compound **[4a]** is formed in a reaction system comprising an alcohol and an alkali metal phosphate, in which the metal (M) is potassium, sodium or lithium; and then compound **[4]** is obtained by acidification of compound **[4a]**.

In the reaction system comprising an alcohol and an alkali metal sulfate, the alkali metal phosphate is tripotassium phosphate, trisodium phosphate, lithium phosphate, etc.; and particularly tripotassium phosphate.

The alcohol used in the reaction is methanol, ethanol or propyl alcohol; and particularly methanol. The reaction temperature is in the range of between 0 and 100°C; and particularly between 20 and 40°C. TLC and HPLC can be used to monitor the progress of the reaction.

A preferable choice of the reaction system is potassium phosphate and methanol at room temperature for 3-6 hours.

(c): In the preparation of compound **[1]** from compound **[4]** or compound **[4a]**, fluorobromomethane first forms a complex with an organic base in a solvent so as to activate the fluoromethyl, and then reacts with compound **[4]** to yield compound **[1]** by fluoromethylation.

The organic base is triethylamine, diethylamine, pyridine, 4-dimethylamino pyridine, α- methylpyridine, etc, all of which have been proven to obtain a satisfying result. The solvent used is a commercially available solvent. In a class of this embodiment, it is a ketone, an alcohol, an ester, an amide, or a halohydrocarbon. The reaction temperature is in a range of between 0 and 30°C. In a class of this embodiment, fluorobromomethane is directly added to the reaction system comprising compound **[4a]** to yield compound **[1]**.

Compound **[2]**, 6α, 9α-difluroro-11β-hydroxy-16α- methyl-17α-propionyloxy-3-oxoandrosta-1,4-diene-17β-carboxylic acid, is obtained by oxidation of flumethasone by periodic acid, and then acylation of the resultant intermediate by propionyl chloride. The synthetic route is shown as follows:

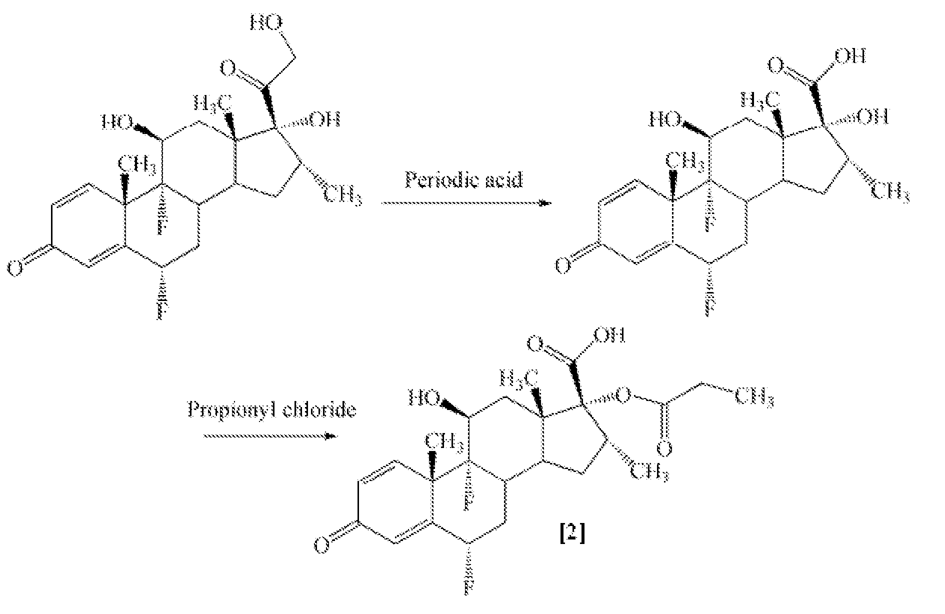

The methods of this invention are carried out under mild reaction conditions to obtain a high quality product. The methods of this invention are suitable for commercial production.

The following examples are intended to show the practice of the invention and are not intended to limit the scope of the present invention.

### EXAMPLES

Example 1

Preparation of 6α,9α-difluroro-11β,17α-dihydroxy-16α-methyl-3-oxoandrosta-1,4-diene-17β-carboxylic acid.

The mixture of 100 g of flumethasone and 500 mL of tetrahydrofuran was cooled to 0°C. A solution of periodic acid (85 g of periodic acid in 250 mL of water) was slowly added and the reaction mixture was allowed to stir for 3 hours. Then, 4 L of water was added, and the system was stirred until a solid had precipitated. The reaction mixture was filtered and the solid was dried to obtain the title compound with a purity higher than 99%.

Example 2

Preparation of 6α,9α-difluroro-11β-hydroxy-16α-methyl-17α-propionyloxy-3-oxoandrosta-1,4-diene-17β-carboxylic acid (Compound **[2]**)

A mixture of 50 g 6α,9α-difluroro-11β,17α-dihydroxy-16α-methyl-3-oxoandrosta-1,4-diene-17β-carboxylic acid obtained in Example 1, 30 g of triethylamine and 800 mL of acetone was stirred and cooled to between -5 and 0°C. 28 g of propionyl chloride was slowly added and stirred for 1 hour, and then 40 mL of diethylamine was added and stirred for 1 hour. The mixture was poured into an acid solution. A solid had precipitated out. The reaction mixture was filtered and the solid was dried to obtain the title compound with a purity of higher than 98%.

Example 3

Preparation of 17β-((N,N-dimethyl-carbamyl)-thio)formoxyl-6α,9α-difluroro-11β-hydroxyl-16α-methyl-17α-propionyloxy-3-oxoandrosta-1,4-diene (Compound **[3]**)

A mixture of 45.5 g of 6α,9α-difluroro-11β-hydroxy-16α-methyl-17α-propionyloxy-3-oxoandrosta-1,4-diene-17β-carboxylic acid, 1 L of butanone, 30.5 g of 4-dimethylamino-pyridine and 24.7 g of N,N-dimethyl thiocarbamoyl chloride was stirred at room temperature for 3 hours and cooled to between 10 and 15°C. 300 mL of DMA and 2 L of water were added. The mixture was cooled to between 0 and 5°C, filtered and washed with water. The resultant solid was dried to obtain the title compound with purity of more than 98.5%.

Example 4

Preparation of 6α,9α-difluroro-11β-hydroxy-16α-methyl-17α-propionyloxy-3-oxoandrosta-1,4-diene-17β- thiocarboxylic acid (Compound **[4]**)

A mixture of 20 g of 17β-((N,N-dimethyl-carbamyl)-thio)formoxyl-6α,9α-difluroro-11β-hydroxyl-16α-methyl-17α-propionyloxy-3-oxoandrosta-1,4-diene, 12.7 g of tripotassium phosphate and 200 mL of methanol was stirred at room temperature for 3 hours under an atmosphere of nitrogen. 200 mL of water were added. The pH value was adjusted to between 1 and 2 with 2N aqueous hydrochloric acid at a temperature controlled below 20°C. The reaction mixture was filtered, and washed with water until the pH was normal. The resultant solid was dried to obtain the title compound with a purity of 98.6%.

Example 5

Preparation of *S*-fluoromethyl-6α, 9α-difluroro-11β-hydroxy-16α-methyl-17α-propionyloxy-3-oxoandrosta-1,4-diene-17β-carbothioate (Compound **[1]**, Fluticasone propionate)

A mixture of 100 mL of acetone and 12.2 g of 4-dimethylamino-pyridine was cooled to between 0 and 5°C. 5.65 g of fluorobromomethane were added and the reaction mixture was stirred for 1 hour. 20 g of 6α,9α-difluroro-11β-hydroxy-16α-methyl-17α-propionyloxy-3-oxoandrosta-1,4-diene-17β-thiocarboxylic acid were added and the reaction mixture was stirred for 2 hours. The mixture was then poured into an acid solution, filtered and washed with water until its pH was neutral. The resultant precipitated solid (fluticasone propionate) was dried. The product was recrystallized from ethyl acetate and a high purity product was obtained. The purity of the product as determined by HPLC was 99.6%.

## Claims

1. A method for preparing fluticasone propionate comprising the following steps:
(1) reacting compound **[2]** with N,N-dimethyl thiocarbamoyl chloride in the presence of a first organic base as catalyst in a ketonic or ethereal solvent at a first reaction temperature to yield compound **[3]**;
(2) decarboxamidating compound **[3]** in a reaction mixture comprising a metal phosphate and an alcohol to yield compound **[4]**; and
(3) reacting fluorobromomethane with a second organic base to form a complex, and then reacting the resulting complex in a second solvent with compound **[4]** to yield compound **[1]**.

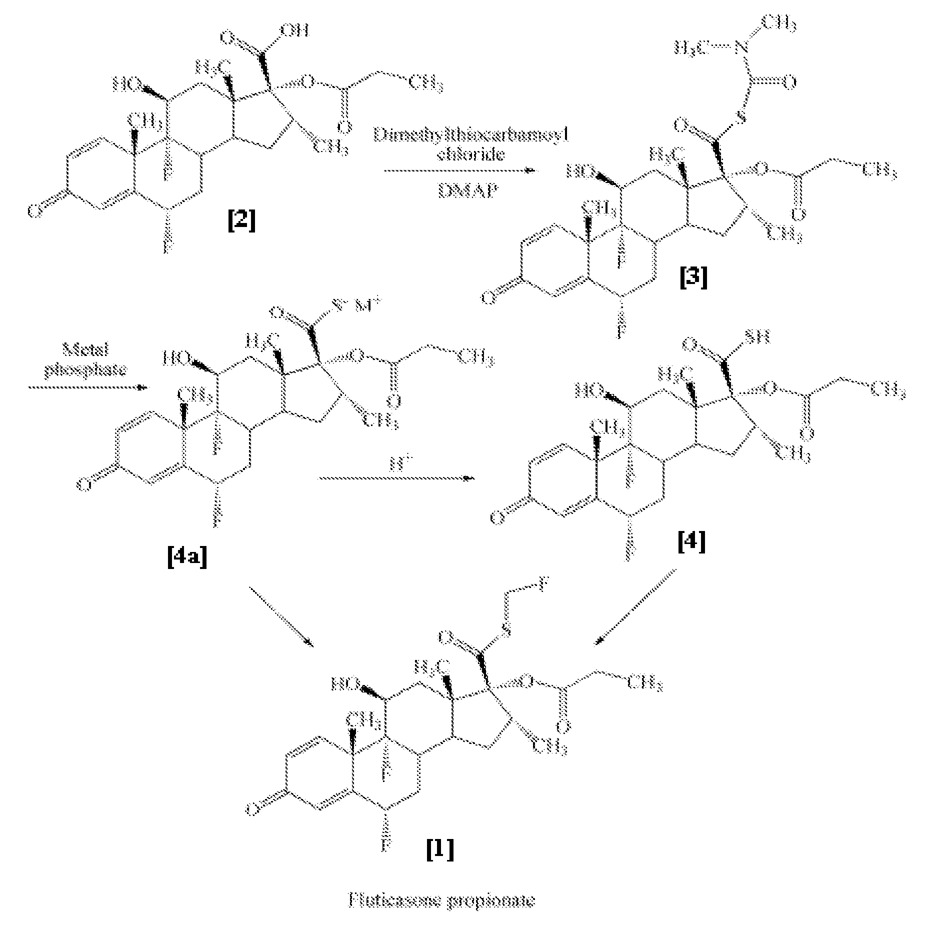

2. A method for preparing fluticasone propionate of claim 1, wherein reacting compound **[2]** with N,N-dimethyl thiocarbamoyl chloride in the presence of a first organic base as catalyst in a ketonic or ethereal solvent at a first reaction temperature to yield compound **[3];** said first organic base is 4-dimethylaminopyridine; the ketonic or ethereal solvent is butanone or tetrahydrofuran; and the reaction temperature is between 0 and 40°C.

3. A method for preparing fluticasone propionate of claim 1, wherein decarboxamidating compound **[3]** in a reaction mixture comprising a metal phosphate and an alcohol at a second reaction temperature to yield compound **[4]**; said metal is potassium, sodium, or lithium; and compound **[4]** is obtained by acidification of an intermediate **[4a]**; said metal phosphate is tripotassium phosphate, trisodium phosphate, or lithium phosphate; said alcohol is methanol, ethanol or propyl alcohol; and the second reaction temperature is between 0 to 100°C,

4. A method for preparing fluticasone propionate of claim 1, wherein reacting fluorobromomethane with a second organic base to form a complex, and then reacting the resulting complex in a second solvent at a third reaction temperature with compound **[4]** or compound **[4a]** to yield compound **[1]**; said second organic base is triethylamine, diethylamine, pyridine, 4-dimethylaminopyridine, or α- methylpyridine; the second solvent is a ketone, an alcohol, an ester, an amide, acetone, DMF or DMA, and the third reaction temperature is between 0 and 30°C.
